# EUROPEAN PATENT SPECIFICATION

(11) **EP 2 371 329 B1**
(45) Date of publication and mention of the grant of the patent: **01.05.2013**
(21) Application number: 11002535.0
(22) Date of filing: 28.03.2011
(51) Int. Cl.: A61F 9/01, A61F 2/14, A61F 9/013

(54) **Method and apparatus for customized preparation of donor corneas for corneal lamellar implant**
Verfahren und Vorrichtung zur maßgeschneiderten Vorbereitung von Spenderhornhaut für eine lamelläre Keratoplastik
Procédé et appareil de préparation personnalisée de cornées de donneur pour implant lamellaire cornéen

(30) Priority: 02.04.2010 IT MI20100571
(43) Date of publication of application: 05.10.2011
(73) Proprietor: IVIS TECHNOLOGIES S.r.l, 74100 Taranto (IT)
(72) Inventor: D' Ippolito, Giuseppe, 74100 Taranto (IT)
(74) Representative: Bruni, Giovanni

(56) References cited:
- EP-A1- 1 462 074
- EP-A2- 1 428 470
- WO-A1-2009/018459
- WO-A2-2007/143111
- US-A1- 2003 060 838
- US-A1- 2009 187 172

## Description

### DESCRIPTION

The present invention relates to a method and an apparatus for customized preparation of donor corneas for corneal lamellar implant.

Preparation of a plurality of corneal implants is disclosed in US2009/0187172A1.

It is known that, in the corneal lamellar implant the donor limb (that is the donor cornel portion to be implanted) has to undergo a series of preparatory steps, in order to be compatible with the morphological features of the receiving structure (that is the eye of the patient the implant is destined to, which is expressly prepared as well). For example, in the endothelial lamellar implant it is used a cutting tool to divide a stromal lamella from an endothelial lamella. The cutting tool can be for example a microkeratome or a femtosecond laser.

The cutting tools carry out a resection of the stromal lamella according to a cutting surface parallel to the outer corneal surface of the donor limb.

In case of endothelial lamellar implant, however, the currently used methods are not able to always guarantee reliable results. In particular, the obtained endothelial lamella has an irregular or uneven profile, with variable and decreasing thickness from the periphery to the centre. Such a profile is not adapted to be integrated with the corneal structure of the receiver, which requires a constant thickness for the whole surface.

In each case, the movement and freedom control in the cutting performance is very limited. As a consequence, the implant efficacy as well, once it is completed, can be compromised and it is very difficult, if not impossible, to prepare the corneal limbs with asymmetric features, which however only in some cases are required.

Therefore, aim of the present invention is to provide a method and an apparatus for the preparation of donor corneas for corneal lamellar implant.

According to the present invention there are provided a method and an apparatus for the preparation of donor corneas for providing corneal lamellar implants as defined in claims 1 and 7, respectively.

The present invention will be described in the following with reference to the appended drawings, which show some examples of not limiting embodiments, in which:
Figure 1 shows a simplified block diagram of an apparatus for the preparation of donor corneas for corneal lamellar implant according to an embodiment of the present invention;
Figure 2 shows a cross section of a donor cornea in an initial step of the method according to the present invention;
Figure 3 shows schematically a pachymetric map of the donor cornea of figure 2;
Figures 4 and 5 show the section of figure 2 in the following steps of the method according to the present invention;
Figure 6 shows the donor cornea of figure 5 divided in a first lamella and a second lamella;
Figure 7 shows a cross section of a cornea where a front lamellar implant was carried out according to a first method;
Figure 8 shows a cross section of a cornea where an endothelial lamellar implant was carried out according to a second method;
Figure 9 shows a flowchart of a method for the treatment of a donor cornea for corneal lamellar implant according to an embodiment of the present invention;
Figure 10 shows a cross section of a cornea of a patient suffering from peripheral keratoconus;
Figure 11 shows a cross section of the cornea of figure 10 treated to receive a donor lamella;
Figure 12 shows a magnified side view of the second lamella of figure 6 modified;
Figure 13 shows a cross section of the cornea of figure 11, where the modified second lamella of figure 12 was implanted;
Figure 14 shows a three-quarter top perspective view of a support for donor lamella preparation;
Figure 15 shows a flowchart of a method for the treatment of a donor cornea for corneal lamellar implant according to an aspect of the present invention.

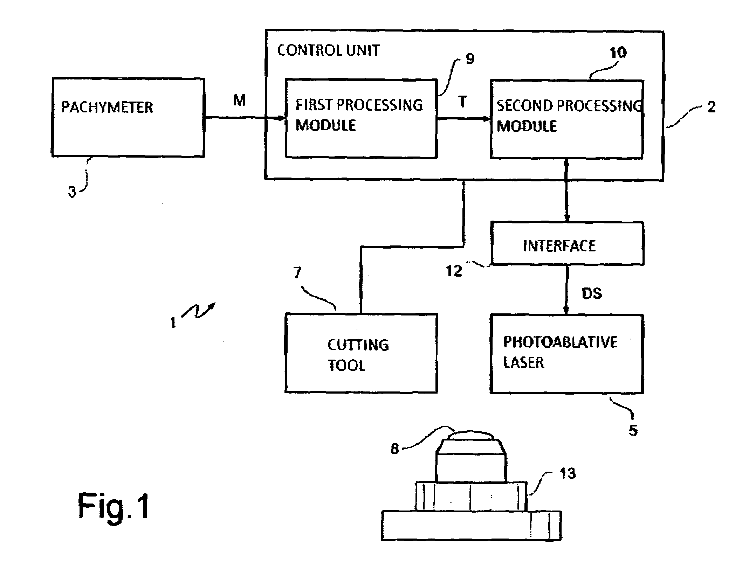

In figure 1, an apparatus for the preparation of donor corneas for corneal lamellar implant is generally indicated with the reference number 1 and comprises a control unit 2, a corneal pachymeter 3, a photoablative laser 5 and a cutting tool 7.

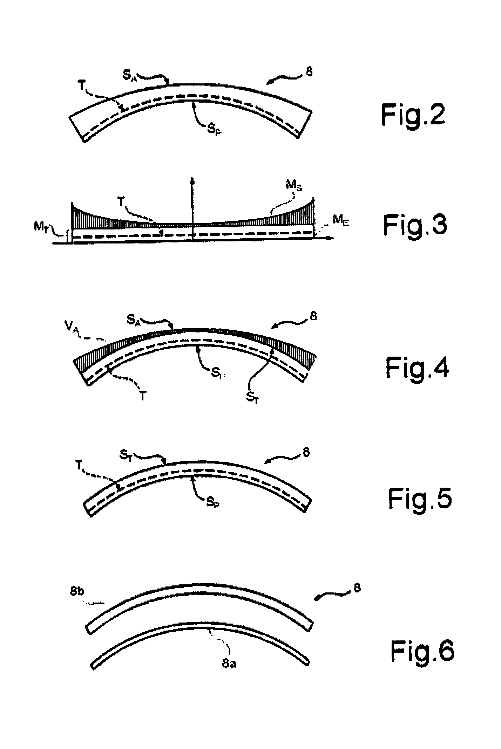

The control unit 2 is coupled with the pachymeter 3, the photoablative laser 5 and the cutting tool 7, so that the bidirectional data exchange and the coordination of the steps for the customized preparation of an explanted limb of a donor cornea (in the following defined as donor limb and indicated with 8 in figures 2-6) are allowed in order to carry out a corneal lamellar implant. In particular, the control unit 2 comprises a first processing module 9, configured to define a cutting surface T (indicated in dotted lines in figure 2) of the donor limb 8, and a second processing module 10, configured to define a target surface S_{T}, which is to be obtained by modifying the front surface S_{A} of the donor limb 8, in order to make the cutting surface T accessible. In an embodiment, the first processing module 9 is provided with a data interface to import a separately defined cutting surface from outside.

The definition of the cutting surface T and of the target surface S_{T} will be described in more details in the following.

The pachymeter 3 allows to detect pachymetric maps M of explanted donor limbs 8. A pachymetric map M is actually a map of the corneal thickness on the whole extension of the cornea or a part thereof (in this case, on the whole extension of the donor limb 8). In order to detect the pachymetric map M, the donor limb can advantageously be applied on an artificial front chamber 13. The artificial front chamber 13 comprises a support provided with adapters (not shown) to receive the donor limb 8 to be prepared, which is maintained in its natural shape. The pachymetric maps M, represented for example in two-dimensional matrix form, having as elements corneal thickness values at respective points of the examined donor limb, are provided to the first processing module 9 of the control unit 2 in order to be further processed to determine the cutting surface T.

For example, the photoablative laser 5 can be an excimer or solid state laser and it is connected to the second module 10 of the control unit 2 by means of a coupling interface 12, which provides DS data arrays in order to carry out the treatment required to obtain the target surface S_{T}. More exactly, the coupling interface 12 reads data from the second module 10 of the control unit 2, concerning the target surface S_{T} and the pachymetry of the donor limb 8 and provides the DS data arrays to the photoablative laser 5, which represent an ablation volume V_{A} (figure 4) which has to be removed from the donor limb 8 by means of photoablation, in order to obtain the target surface S_{T} defined by the second processing module 10. According to the DS data arrays, the photoablative laser 5 carries out the photoablation of the ablation volume V_{A}.

In an embodiment, the interface 12 is mounted in the control unit 2, for example in the second processing module 10.

The cutting tool 7 is a very high precision tool, for example a microkeratome or a femtosecond laser. The cutting tool 7 is guided by an inner control unit (not shown) or, in alternative, directly by the control unit 2 to carry out a cut of the donor limb 8 according to the cutting surface T. As shown in figures 5 and 6, the cut is carried out at constant depth from the front surface of the donor limb 8 along the cutting surface T, and divides the treated donor limb 8 in an endothelial lamella 8a and in a stromal lamella 8b, both used for implant in different patients.

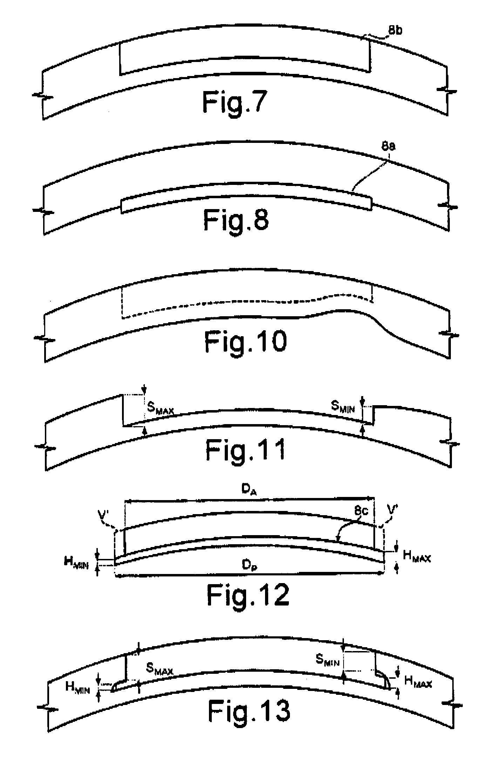

The endothelial lamella 8a is ready to be implanted in the receiving bed, as is shown in figure 8 (endothelial corneal implant). The stromal lamella 8b can be used as it is (figure 7, front lamellar implant/epikeratophakia) or with other possible variations according to the kind of pathology of the receiving patient, as is explained in the following.

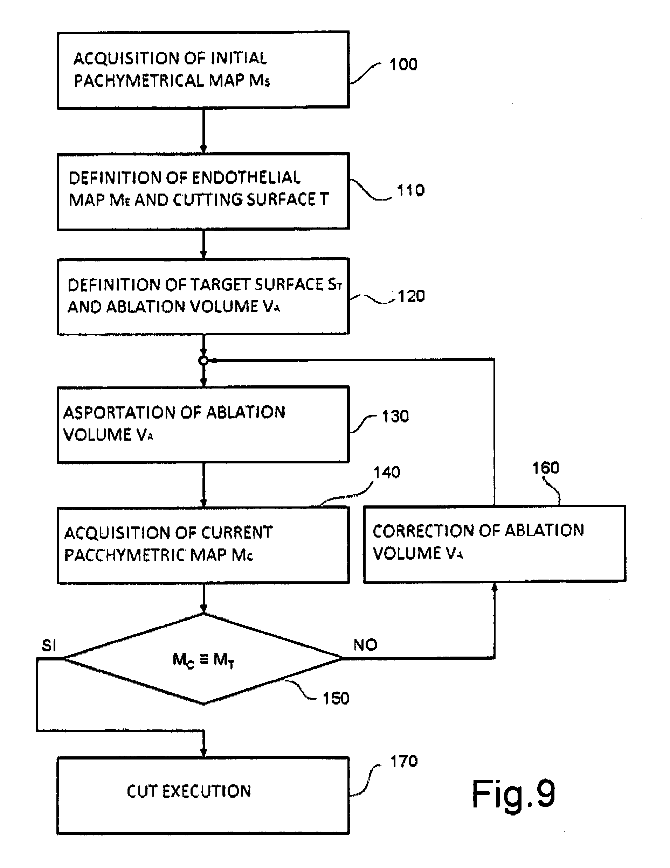

In figure 9, it is shown a flowchart concerning a method for the treatment of a donor cornea for corneal lamellar implant, which is carried out by means of the apparatus 1. In the example here described, the method is applied to the donor limb 8, the figure 2 showing a cross section thereof after the explant, before the treatment.

At the beginning (figure 9, block 100), an initial pachymetric map Mₛ of the donor limb 8 is acquired. Here and in the following, it is intended that the initial pachymetric map Mₛ can be acquired by means of direct instrumental detection of the donor limb 8, both by collection or storage withdrawal (for example incorporated in the control unit 2 and here not shown or a removable data medium, as for example an optical or magnetic disk) or by receiving (for example by means of a transmission line) representative data of a direct instrumental detection of the donor limb 8, both by means of collection or storage withdrawal or by receiving representative data of a standard pachymetric map defined from data relating to statistically normal organs. In the embodiment here described, in particular, the initial pachymetric map Mₛ is acquired by means of the pachymeter 3. As just said, the initial pachymetric map Mₛ detected by the pachymeter 3 is provided in form of a two-dimensional matrix. For easiness of description, figure 3 shows a pachymetric profile obtained from the initial pachymetric map Mₛ and corresponding to the cross section of figure 2 of the donor limb 8. The initial pachymetric map M_{S} of the donor limb 8 is provided to the first and second processing module 9, 10 of the control unit 2.

The cutting surface T is defined by means of the first processing module 10 (figure 9, block 110; see also figures 2-5). In practice, an endothelial pachymetric map M_{E} is determined and stored in the first processing module 9. The endothelial pachymetric map M_{E} represents locally the desired thickness of the endothelial lamella 8a to be realized by cutting the donor limb 8 (for example a constant thickness map). The cutting surface T is a surface shaped so that, with the rear surface S_{P} of the donor limb 8, the desired thickness for the endothelial lamella 8a is defined according to the endothelial pachymetric map M_{E}. In other words, the distance between the rear surface Sp of the donor limb 8 and the cutting surface T results, in each point, from the corresponding value of the endothelial pachymetric map M_{E}. In an embodiment, the cutting surface T is defined so that the endothelial lamella 8a has a constant thickness. For example, the rear surface S_{P} of the donor limb 8 and the cutting surface T are concentric spherical surfaces, respectively with lower and greater radius and the difference between the radii is equal to the desired thickness for the endothelial lamella 8a.

Once the cutting surface T to be realized is defined, by means of the second processing module 10 of the control unit 2, is determined the target surface S_{T} in order to make the cut accessible along the same cutting surface T (figure 9, block 120; see also figure 4, where it is shadowed the ablation volume V_{A} comprised between the front surface of the limb S_{A} and the target surface S_{T}). The target surface S_{T} considers the operative features of the cutting tool 7 provided in the apparatus 1, in particular the fact that the cut is carried out at a constant depth with respect to the free surface of the donor limb 8.

More in detail, together with the cutting surface T the target surface S_{T} identifies a region with constant thickness, which in practice corresponds to the stromal lamella 8b to be obtained by cutting the donor limb 8. At the target surface S_{T} corresponds a target pachymetric map M_{T} defined between the same target surface S_{T} and the rear surface S_{P} of the donor limb 8. In an embodiment, the target surface S_{T} and the cutting surface T are concentric. The ablation volume V_{A} (figure 4) is comprised between the front surface S_{A} of the donor limb 8 and the target surface S_{T}. In the not limiting embodiment here described, the ablation volume V_{A} is determined on the basis of the difference between the initial pachymetric map M_{S} and the target pachymetric map M_{T}. The ablation volume V_{A}, in particular, can be determined by the second processing module 10, by the interface 12 or by any other component (not shown) of the control unit 2.

Subsequently (figure 9, block 130), the photoablative laser 5 is guided through the coupling interface 12 so that the ablation volume V_{A} is removed and the target surface S_{T} is obtained. The corneal limb 8 is thus shaped and shaped similar to the cutting surface T.

In an embodiment, it is therefore carried out a pachymetric test of the result obtained. In detail, it is acquired a current pachymetric map M_{C} (figure 9, block 140) and it is compared with the target pachymetric map M_{T} (block 150).

If the current pachymetric map M_{C} and the target pachymetric map M_{T} do not correspond (that is if the current thickness of the donor limb 8 does not correspond locally point to point to the distance provided between the target surface S_{T} and the rear surface S_{P}; block 150, exit NO), the ablation volume V_{A} is corrected (block 160) and a new ablation is carried out (block 130).

On the contrary, that is if the target surface S_{T} is obtained and the current pachymetric map M_{C} corresponds to the target pachymetric map M_{T} (block 150, exit SI), the corneal limb 8 is cut (block 170; see also figure 6) and is divided in the endothelial lamella 8a and the stromal lamella 8b. The described method and apparatus allow advantageously to carry out accurately the preparation and the cut according to the desired cutting surface. Moreover, in general, if the cutting performance is controlled in a better way, it is possible to follow non-spherical and however arbitrarily shaped profiles, according to the precision allowed by the cutting tools.

In an alternative embodiment, the ablation volume V_{A} is determined without using the pachymeter 3. It is to be observed in fact that the donor lamellae, since necessarily coming from healthy organs, have limited variability. It is therefore possible to provide standard pachymetric maps from data relating to statistically normal organs. A standard pachymetric map can be stored in the control unit 2 and acquired by the second processing module 10 (or by the interface 12 or any other component of the control unit 2) to define the ablation volume V_{A}. In particular, the ablation volume V_{A} is determined from the difference between the acquired standard pachymetric map and the target pachymetric map M_{T} (defined on the basis of the receiver needs as described) . Even if it is not possible to obtain the same precision provided by a pachymeter, by using the standard pachymetric maps there are produced normally acceptable results and the result of the implant surgery is not compromised.

As previously stated, the stromal lamella 8b can need other treatments according to the features of the receiving bed.

For example, figure 10 shows the case of a patient suffering from keratoconus localized in a peripheral region of the cornea. According to a method of treatment, a thin receiving bed with uniform thickness is arranged by means of asymmetric ablation of corneal material (figure 10, dotted line). Therefore it is modified the stress state of the residual material, which tends to stretch thus recovering the irregularities due to the presence of the keratoconus (figure 11). If the keratoconus is in a strongly decentralized position, the asymmetric treatment can produce a receiving bed with margins of different height (comprised between a maximum height S_{MAX} and a minimum height S_{MIN}, figure 11) and cause problems during cicatrization.

More generally, the problem can arise also when the margin height of the receiving bed is uniformly too thin. In such a case, the margin of the lamella to be implanted is in fact usually introduced in a task obtained by means of a recessed circular cut ("undercut") at the bottom of the receiving bed. However, the thickness of the lamella introduced in the task can lead to too marked discontinuities to be reabsorbed without consequences in the cicatrization process.

In order to obviate such drawbacks, it is realized a stromal lamella 8b with greater diameter than the diameter of the receiving bed. The stromal lamella 8b is further shaped by means of a photoablative treatment, in which a volume of tissue is removed on the margin of the stromal lamella 8b, so that a peripheral edge 8c with controlled thickness which does not extend on the whole perimeter, is provided at the base of the stromal lamella 8b. Therefore the stromal lamella 8b has a front diameter D_{A} lower than a rear diameter D_{P} (figure 12).

The peripheral edge 8c can be shaped so that it has a constant thickness (when the margins of the receiving bed do not have appreciable height variations) or a variable one (on the contrary, for example for patients suffering from peripheral keratoconus).

In the second case, the peripheral edge 8c of the stromal lamella 8b is asymmetrically shaped (figure 12), complementarily to the thickness at the margins of the receiving bed. In other words, the peripheral edge 8c of the stromal lamella 8b is shaped so that it has a variable thickness between a maximum H_{MAX} and a minimum H_{MIN}, which are determined on the basis of the features of the receiving bed. In particular, the volume to be removed is determined in such a way that the sum of the thickness of the peripheral edge 8c and the height of the margin of the receiving bed is substantially constant along the whole perimeter.

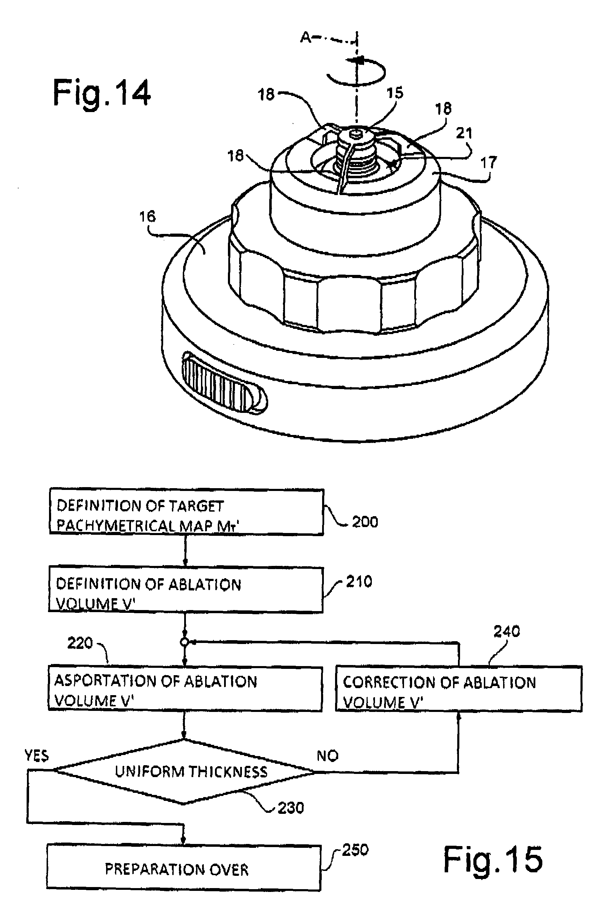

In this step, the photoablation is carried out by means of a rotating mask 15, shown in figure 14, which is mounted on a fixed support 16. More in detail, the mask 15 is shaped as a disk and is provided with a coaxial annular base 17, to which it is connected by means of arms 18. The base 17 is rotatably connected to the support 16 and is actuated by means of an inner driving member (not shown), such that the mask 15 rotates about a central axis A perpendicular to a face of the mask 15 and coaxially to the fixed support 16. Inside the fixed support 16, it is defined a recess 21 to receive the stromal lamella 8b coaxially to the mask 15, which is suspended on the recess 21 by means of the arms 18. During the photoablation, the stromal lamella 8b is thus partially shielded by the mask 15 and its central portion is effectively protected.

By using the rotating mask 15 and the support 16 it is also guaranteed the self-centering of the treatment with respect to the stromal lamella 8b and localized shadow effects are prevented, thanks to the rotating arms 18.

It is therefore carried out a circular incision at the bottom of the receiving bed, so that the diameter of the bottom is widened without additional removal of tissue (figure 13). The stromal lamella 8b is then accommodated in the receiving bed, with the peripheral edge 8c introduced in the circular incision. In particular, the thicker portion of the peripheral edge 8c is at the thinnest margin of the receiving bed, and vice versa, the thinnest portion of the peripheral edge 8c is at the thicker margin of the receiving bed. In this way, the sum of the thickness of the peripheral edge 8c and the height of the margin of the receiving bed is substantially constant along the whole perimeter.

In order to realize the desired peripheral edge 8c (figure 12), it is defined a new target pachymetric map M_{T}', in which the peripheral edge corresponds to the desired edge (figure 15, block 200).

On the basis of the target pachymetric map M_{T}' , it is defined a new ablation volume V', which allows to remove the tissue in excess from the desired profile from the stromal lamella 8b (block 210).

By using the mask 15 mounted on the support 16, the ablation volume V' is removed from the corneal limb 8, which is shaped so that the desired profile is obtained (block 220). It is thus carried out a pachymetric test of the result obtained from the ablation of the volume ablation V' (block 230).

If the current thickness of the donor limb 8 does not correspond to the target pachymetric map M_{T}' (block 230, exit NO), the ablation volume V' is corrected (block 240) and it is carried out a new ablation (block 220).

On the contrary, that is if it is obtained the desired profile (block 230, exit SI), the procedure for preparing the donor limb ends (block 250).

It is eventually clear that changes and modifications can be made to the described apparatus without departing from the scope of the present invention, as defined in the appended claims.

## Claims

1. Method for preparation of a donor cornea for corneal transplantation, comprising the following steps:
acquiring (100) an initial pachymetric map (Mₛ);
defining (110) a cutting surface (T) of an explanted donor cornea (8);
defining a target surface (S_{T}) on the basis of the cutting surface (T);
defining a target pachymetric map (M_{T}) comprised between the target surface (S_{T}) and a rear surface (S_{P}) of the donor cornea (8);
defining an ablation volume (V_{A}) on the basis of the difference between the initial pachymetric map (M_{S}) and the target pachymetric map (M_{T});
shaping (130), by removing the ablation volume (V_{A}), a front surface (S_{A}) of the donor cornea (8) so as to obtain the target surface (S_{T});
dividing (170) the donor cornea (8) along the cutting surface (T) in a first lamella (8a), defined between said rear surface (S_{P}) and the cutting surface (T), and a second lamella (8b) defined between the target surface (S_{T}) and the cutting surface (T).

2. Method according to claim 1, wherein the step of acquiring (100) an initial pachymetric map (M_{S}) comprises detecting the initial pachymetric map (M_{S}) of the donor cornea (8) by means of a pachymeter; or receiving a standard pachymetric map defined on the basis of data relating to statistically normal organs.

3. Method according to claim 1 or 2, wherein the target surface (S_{T}) is determined so that a region with constant thickness is defined between the target surface (S_{T}) and the cutting surface (T).

4. Method according to any one of the preceding claims, wherein the step of acquiring (110) the cutting surface (T) comprises determining an endothelial pachymetric map (M_{E}) which represents a desired local thickness of the first lamella (8a).

5. Method according to any one of the preceding claims, comprising the following steps :
acquiring (140) a current pachymetric map (M_{C}) after the step of shaping the front surface (S_{A}) of the donor cornea (8);
comparing (150) the current pachymetric map (M_{C}) with the target pachymetric map (M_{T});
further shaping (130) the donor cornea (8) if the current pachymetric map (M_{C}) and the target pachymetric map (M_{T}) do not match; and
repeating acquiring (140) a current pachymetric map (M_{C}), comparing (150) and further shaping (130) until the current pachymetric map (M_{C}) and the target pachymetric map (M_{T}) match.

6. Method according to any one of the preceding claims, comprising the step of asymmetrically shaping a peripheral edge (8c) of the second lamella (8b), so that a thickness of the peripheral edge (8c) varies between a maximum (H_{MAX}) and a minimum (H_{MIN}), preferably so that the sum of the thickness of the peripheral edge (8c) and of a height of a margin of a receiving bed intended to receive the donor cornea (8) is substantially constant along the perimeter of the donor cornea (8).

7. Apparatus for preparation of a donor cornea for corneal transplantation, comprising:
a control unit (2) configured to define a cutting surface (T) of an explanted donor cornea (8) to be prepared for the transplantation and a target surface (S_{T}) of the rear surface (S_{P}) of the donor cornea (8) on the basis of the cutting surface (T);
the control unit (2) being further configured to receive an initial pachymetric map (M_{S}) to define a target pachymetric map (M_{T}) comprised between the target surface (S_{T}) and the rear surface (S_{P}) and to define, on the basis of the difference between the initial pachymetric map (M_{S}) and the target pachymetric map (M_{T}), a first ablation volume (V_{A}) to be removed from the donor cornea (8) to obtain the target front surface (S_{T});
a corneal pachymeter (3) configured to acquire the initial pachymetric map (M_{S}) from the donor cornea (8) and coupled with the control unit (2) to provide the initial pachymetric map (M_{S});
a photoablative laser (5) for shaping the explanted donor cornea (8);
a cutting tool (7) for dividing (170) the donor cornea (8) along the cutting surface (T) in a first lamella (8a) defined between a rear surface (S_{P}) of the donor cornea (8) and the cutting surface (T), and a second lamella (8b) defined between the cutting surface (T) and the target surface (S_{T});
wherein the photoablative laser (5) is coupled with the control unit (2)and is controllable to shape a front surface (SA) of the donor cornea by removing the first ablation volume (VA) in order to obtain the target surface (ST).

8. Apparatus according to claim 7, wherein the control unit (2) is configured to define a desired profile for an edge (8c) of the second lamella (8b), to determine a second ablation volume (V') to be removed from the second lamella (8b) in order to obtain the desired profile for the edge (8c) of the second lamella (8b) and to provide representative data of the second ablation volume (V') to the photoablative laser (5).

9. Apparatus according to claim 7 or 8, comprising a mask (15) shaped as a disk and provided with a coaxial annular base (17), to which the mask (15) is connected by means of arms (18);
wherein the base (17) is rotatably coupled with a fixed support (16) to be rotated by a driving member, so that the mask (15) rotates about an axis (A) perpendicular to a face of the mask (15) and passing through the centre of the mask (15);
and wherein a recess (21) is provided inside the support (16) in order to accommodate the donor cornea (8) coaxially to the mask (15), wherein the arms (18) are configured to suspend the mast (15) onto the recess (21).

## Patentansprüche

1. Vorfahren zum Vorbereiten einer Spenderhornhaut für eine Hornhauttransplantation das die folgenden Schritte umfasst
Erfassen (100) einer Ausgangspachymetriekarte (Mₛ),
Definieren (110) einer Schneidfläche (T) einer explantierten Spendorhornhaut (8),
Definieren einer Zieloberfläche (S_{T}) auf Grundlage der Schneidfläche (T),
Definierten einer Zielpachymetriekarte (M_{T}) die zwischen der Zieloberfläche (S_{T}) und einer hinteren Oberfläche (S_{P}) der Spenderhornhaut (8) eingeschlossen ist;
Definieren eines Ablationsvolumens (V_{A}) auf Grundlage dos Unterschieds zwischen der Ausgangspachymetriekarte (M_{S}) und der Zielpachymetriakarte (M_{T})
Formen (130) durch Abtragen des Ablationsvolumens (V_{A}) einer vorderen Oberfläche (S_{A}) der Spenderhornhaut (8), um die Zieloberfläche (S_{T}) zu erhalten,
Teilen (170) der Spenderhornhaut (S) entlang der Schneidfläche (T) in eine erste Lamelle (8a), die zwischen der hinteren Oberfläche (S_{P}) und der Schneidfläche (T) definiert ist, und eine zweite Lamelle (8b), die zwischen der Zieloberfläche (S_{T}) und der Schneidfläche (T) definiert ist

2. Verfahren nach Anspruch 1 bei dem der Schritt des Erfassens (100) einer Ausgangspachymetriekarte (M_{S}) das Detektieren der Ansgangspachymetriekarte (M_{S}) der Spenderhornhaut (8) mit Hilfe eines Pachymeters oder das Empfangen einer Standardpachymetriekarte umfasst, die auf Grundlage von Daten definiert wurde die sich auf statistisch normale Organe beziehen.

3. Verfahren nach Anspruch 1 oder 2, bei dem die Zieloberfläche (S_{T}) so bestimmt wird, dass eine Region mit konstanter Dicke zwischen der Zieloberfläche (S_{T}) und der Schneidfläche (T) definiert ist.

4. Vorfahren nach einem der vorhergehenden Ansprüche, bei dem der Schritt der Erfassens (110) der Schneidfläche (T) das Bestimmen einer Endothelpachymetriekarte (M_{E}) umfasst, die eine gewünschte lokale Dicke der ersten Lamelle (8a) darstellt

5. Verfahren nach einem der vorhergehenden Ansprüche, das die folgenden Schritte umfasst
Erfassen (140) einer aktuellen Pachymetriekarte (M_{C}) nach dem Schritt das Formens der vorderen Oberfläche (S_{A}) der Spenderhornhaut (8);
Vergleichen (150) der aktuellen Pachymetriekarte (M_{C}) mit der Zielpachymetriekarte (M_{T});
weiteres Formen (130) der Spenderhornhaut (8), wenn die aktuelle Pachymetriekarte (M_{C}) und die Zielpachymetriekarte (M_{T}) nicht übereinstimmen, und
Wiederholen des Erfassens (140) einer aktuellen Pachymetriekarte (M_{C}), Vorgleichen (150) und weiteres Formen (130), bis die aktuelle Pachymetriekarte (M_{C}) und die Zielpachymetriekarte (M_{T}) übereinstimmen

6. Verfahren nach einem der vorhergehenden Ansprüche, das den Schritt des asymmetrischen Formens eines Umfangsrands (8c) der zweiten Lamelle (8b) derart umfasst, dass eine Dicke des Umfangsrands (8c) zwischen einem Maximum (H_{MAX}) und einem Minimum (H_{MIN}) vorzugsweise so variiert, das die Summe aus der Dicke des Umfangsrands (8c) und einer Höhe eines Rands eines für die Aufnahme der Spenderhornhaut (8) vorgesehenen Empfängerbetts im Wesentlichen entlang dem Umfang der Spenderhornhaut (8) konstant ist

7. Vorrichtung zum Vorbereiten einer Spenderhornhaut für die Hornhauttransplantation, umfassend:
eine Steuereinheit (2), die gestaltet ist, um eine Schneidfläche (T) einer für die Transplantation vorzubereitenden explantierten Spenderhornhaut (8) und eine Zieloberfläche (S_{T}) der hinteren Oberfläche (S_{P}) der Spenderhornhaut (8) auf Grundlage der Schneidfläche (T) zu definieren,
wobei die Steuereinheit (2) ferner gestaltet ist, um eine Ausgangspachymetriekarte (M_{S}) zu empfangen, um eine Zielpachymetriekarte (M_{T}) zu definieren, die zwischen der Zieloberfläche (S_{T}) und der hinteren Oberfläche (S_{P}) eingeschlossen ist, und um auf Grundlage des Unterschieds zwischen der Ausgangschymetriekarte (M_{S}) und der Zielpachymetriekarte (M_{T}) ein erstes Ablationsvolumen (V_{A}) zu definieren, das von der Spenderhornhaut (8) abzutragen ist, um die angezielte vordere Oberfläche (S_{T}) zu erhalten,
ein Hornhautpachymeter (3), das gestaltet ist, um die Ausgangspachymetriekarte (M_{S}) von der Spenderhornhaut (8) zu erfassen, und mit der Steuereinheit (2) verbunden ist, um die Ausganspachymetriekarte (M_{S}) bereitzustellen,
einen photoablativen Laser (5) zum Formen der explantierten Spenderhornhaut (8),
ein Schneidwerkzeug (7) zum Teilen (170) der Spenderhornhaut (8) entlang der Schneidfläche (T) in eine erste Lamelle (8a), die zwischen einer hinteren Oberfläche (S_{P}) der Spenderhornhaut (8) und der Schneidfläche (T) definiert ist, und eine zweite Lamelle (8b), die zwischen der Schneidfläche (T) und
der Zieloberfläche (S_{T}) definiert ist,
wobei der photoablative Laser (5) mit der Steuereinheit (2) verbunden ist und gesteuert werden kann, um eine vordere Oberfläche (S_{A}) der Spenderhornhaut durch Abtragen des ersten Ablationsvolumens (V_{A}) zu formen, um die Zieloberfläche (S_{T}) zu erhalten

8. Vorrichtung nach Anspruch 7, bei der die Steuereinheit (2) gestaltet ist, um ein gewünschtes Profil für einen Rand (8c) der zweiten Lamelle (8b) zu definieren, um ein zweites Ablationsvolumen (V') zu definieren, das von der zweiten Lamelle (8b) abzutragen ist, um das gewünschte Profil für den Rand (8c) der zweiten Lamelle (8b) zu erhalten, und um dem photoablativen Laser (5) repräsentative Daten des zweiten Ablationsvolumens (V') bereitzustellen.

9. Vorrichtung nach Anspruch 7 oder 8, die eine Maske (15) umfasst, die als eine Scheibe ausgeformt und mit einer koaxialen ringförmigen Basis (17) versehen ist, mit der die Maske (15) mit Hilfe von Armen (18) verbunden ist,
wobei die Basis (17) drehbar mit einem festen Support (16) verbunden ist, der von einem Antriebselement gedroht wird, so dass sich die Maske (15) um eine Achse (A) dreht, die perpendikular zu einer Seile der Maske (15) ist und durch den Mittelpunkt der Maske (15) verläuft,
und wobei eine Aussparung (21) in dem Support (16) vorgesehen ist, um die Spenderhornhaut (8) koaxial zur Maske (15) unterzubringen, wobei die Arme (18) dazu ausgestaltet sind, die Maske (15) Über der Aussparung (21) aufzuhängen

## Revendications

1. Procédé pour la préparation d'une cornée de donneur pour une greffe de la cornée, comprenant les étapes suivantes :
l'acquisition (100) d'une carte pachymétrique initiale (Mₛ) ;
la définition (110) d'une surface de coupe (T) d'une cornée de donneur explantée (8) ;
la définition d'une surface cible (S_{T}) sur la base de la surface de coupe (T) ;
la définition d'une carte pachymétrique cible (M_{T}) comprise entre la surface cible (S_{T}) et une surface postérieure (S_{P}) de la cornée de donneur (8) ;
la définition d'un volume d'ablation (V_{A}) sur la base de la différence entre la carte pachymétrique initiale (Mₛ) et la carte pachymétrique cible (M_{T}) ;
le modelage (130), en retirant le volume d'ablation (V_{A}), d'une surface antérieure (S_{A}) de la cornée de donneur (8) de manière à obtenir la surface cible (S_{T});
la division (170) de la cornée de donneur (8) le long de la surface de coupe (T) en une première lamelle (8a), définie entre ladite la surface postérieure (S_{P}) et la surface de coupe (T), et une deuxième lamelle (8b) définie entre la surface cible (S_{T}) et la surface de coupe (T).

2. Procédé selon la revendication I, dans lequel l'étape d'acquisition (100) d'une carte pachymétrique initiale (Mₛ) comprend :
la détection de la carte pachymétrique initiale (Mₛ) de la cornée de donneur (8) au moyen d'un pachymètre ; ou
la réception d'une carte pachymétrique standard définie sur la base de données relatives à des organes statistiquement normaux.

3. Procédé selon la revendication 1 ou 2, dans lequel la surface cible (S_{T}) est déterminée de manière qu'une région avec une épaisseur constante soit définie entre la surface cible (S_{T}) et la surface de coupe (T).

4. Procédé selon l'une quelconque des revendications précédentes, dans lequel l'étape d'acquisition (110) de la surface de coupe (T) comprend la détermination d'une carte pachymétrique endothéliale (M_{E}) qui représente une épaisseur locale désirée de la première lamelle (8a).

5. Procédé selon l'une quelconque des revendications précédentes, comprenant les étapes suivantes :
l'acquisition (140) d'une carte pachymétrique actuelle (M_{C}) après l'étape de modelage de la surface antérieure (SA) de la cornée de donneur (8) ;
la comparaison (150) de la carte pachymétrique actuelle (M_{C}) avec la carte pachymétrique cible (M_{T}) ;
le modelage ultérieur (130) de la cornée de donneur (8) si la carte pachymétrique actuelle (M_{C}) et la carte pachymétrique cible (M_{T}) ne coïncident pays ; et
la répétition de l'acquisition (140) d'une carte pachymétrique actuelle (M_{C}), la comparaison (150) et le modelage ultérieur (130) jusqu'à ce que la carte pachymétrique actuelle (M_{C}) et la carte pachymétrique cible (Mᵣ) coïncident.

6. Procédé selon l'une quelconque des revendications précédentes, comprenant l'étape consistant à modeler asymétriquement un bord périphérique (8c) de la deuxième lamelle (8b), de manière qu'une épaisseur du bord périphérique (8c) varie entre un maximum (H_{MAX}) et un minimum (H_{MIN}), de préférence de manière que la somme de l'épaisseur du bord périphérique (8c) et de la hauteur d'une marge d'un lit de réception destiné à recevoir la cornée de donneur (8) soit sensiblement constante le long du périmètre de la cornée de donneur (8).

7. Dispositif pour la préparation d'une cornée de donneur pour une greffe de la cornée, comprenant :
une unité de commande (2) configurée pour définir une surface de coupe (T) d'une cornée de donneur explantée (8) à préparer pour la greffe et une surface cible (S_{T}) de la surface postérieure (S_{P}) de la cornée de donneur (8) sur la base de la surface de coupe (T) ;
l'unité de commande (2) étant configurée en outre pour recevoir une carte pachymétrique initiale (M_{S}) pour définir une carte pachymétrique cible (M_{T}) comprise entre la surface cible (S_{T}) et la surface postérieure (S_{P}) et pour définir, sur la base de la différence entre la carte pachymétrique initiale (M_{S}) et la carte pachymétrique cible (M_{T}), un premier volume d'ablation (V_{A}) à enlever de la cornée de donneur (8) pour obtenir la surface antérieure cible (S_{T}) ;
un pachymètre cornéen (3) configuré pour acquérir la carte pachymétrique initiale (M_{S}) à partir de la cornée de donneur (8) et couplé avec l'unité de commande (2) pour fournir la carte pachymétrique initiale (M_{S}) ;
un laser photoablatif (5) pour modeler la cornée de donneur explantée (8);
un outil de coupe (7) pour diviser (170) la cornée de donneur (8) le long de la surface de coupe (T) en une première lamelle (8a) définie entre une la surface postérieure (S_{P}) de la cornée de donneur (8) et la surface de coupe (T), et une deuxième lamelle (8b) définie entre la surface de coupe (T) et la surface cible (S_{T}) ;
dans lequel le laser photoablatif (5) est couplé avec l'unité de commande (2) et est contrôlable pour modeler une surface antérieure (SA) de la cornée de donneur en enlevant le premier volume d'ablation (VA) de manière à obtenir la surface cible (ST).

8. Dispositif selon la revendication 7. dans lequel l'unité de commande (2) est configurée pour définir un profit désiré pour un bord (8c) de la deuxième lamelle (8b), pour déterminer un deuxième volume d'ablation (V') à enlever de la deuxième lamelle (8b) de manière à obtenir le profil désiré pour le bord (8c) de la deuxième lamelle (8b) et pour fournir des données représentatives du deuxième volume d'ablation (V') au laser photoablatif (5).

9. Dispositif selon la revendication 7 ou 8, comprenant un masque (15) profilé comme un disque et muni d'une base annulaire coaxiale (17), à laquelle le masque (15) est connecté au moyen de bras (18) ;
dans lequel la base (17) est couplée de manière rotative avec un support fixe (16) pour être déplacée par rotation par un élément d'entraînement, de manière que le masque (15) tourne autour d'un axe (A) perpendiculaire à une face du masque (15) et passant à travers le centre du masque (15) ;
et dans lequel un évidement (21) est prédisposé à l'intérieur du support (16) de manière à loger la cornée de donneur (8) de manière coaxiale avec le masque (15), dans lequel les bras (18) sont configurés pour suspendre le masque (15) sur l'évidement (21).
